# EUROPEAN PATENT SPECIFICATION

(11) **EP 0 437 999 B1**
(45) Date of publication and mention of the grant of the patent: **13.07.1994**
(21) Application number: 90403731.4
(22) Date of filing: 21.12.1990
(51) Int. Cl.: B29C 65/40, B29C 65/00, B29C 43/28, B32B 3/00, A47G 9/02

(54) **A manufacturing method of a magnetic sheet**
Verfahren zur Herstellung eines magnetischen Bettuchs
Procédé de fabrication d'un drap magnétique

(30) Priority: 18.01.1990 JP 10178/90
(43) Date of publication of application: 24.07.1991
(73) Proprietor: NIHONKENKOZOSHINKENKYUKAI CO. LTD., Fukuoka-shi Fukuoka 813 (JP)
(72) Inventor: Masuda, Isamu, Chuo-ku, Fukuoka-shi, Fukuoka 814-01 (JP)
(74) Representative: Bertrand, Didier

(56) References cited:
- DE-A- 3 041 965
- DE-A- 3 522 667
- FR-A- 2 013 239
- FR-A- 2 611 306

## Description

The present invention relates mainly to the technical field of bedding goods such as a magnetic bedspread and a magnetic mattress for health promotion purpose. Particularly, it relates to a manufacturing method of a magnetic sheet to be filled into bedding goods of this kind.

In the past, for magnetic sheets of this kind, such sheets having mounted a multiple number of plastic magnets on the surface of the sheet material such as a cushioning material or cloth have been offered in the market.

For mounting these plastic magnets on the sheet, a known method uses adhesives on the surface of the sheet material. According to another method, a stopper stem is inserted into a dented hole of each plastic magnet from the other side of the sheet material and then fixed.

In the case of the former method, plastic magnets tend to fall off from the sheet material, causing to shorten the life of the product.

In the case of the latter method using stoppers, the stopper stems are often broken or come off from the dented hole, causing the magnets to come off. Moreover, stoppers add weight to the sheet, making it difficult to decrease the weight of magnetic bedspread.

This invention was made with these problems in view, and offers a manufacturing method of a magnetic sheet that can avoid plastic magnets from falling off and helps in reducing weight of the magnetic sheet.

The present invention relates to a manufacturing method of a magnetic sheet whereby a multiple number of plastic magnets are mounted on the surface of a sheet material. Said manufacturing comprises a first step in which the fixing surface of the plastic magnets is subject to processing with a chemical so that it is eroded to be roughened to expose the magnetic powder particles, a second step in which the roughened surface of each magnet is coated with hot-melt adhesives, and a third step in which the sheet material and each magnet are heat-pressed to be bonded after applying the adhesive-coated surface of each magnet on the sheet material.

In accordance with the invention, with the plastic magnet having formed a roughened surface, the area coated with hot-melt adhesives is increased to a large extent. Moreover, the adhesives penetrate amply into intricately roughened surface portions. For this reason, the bonding strength of the adhesives with the plastic magnets is extremely great, thus preventing any risk of falling off for the plastic magnets. With no extra means such as stoppers used, contributing to render the product made light in weight, the invention assure that a magnetic sheet thus made can be well used in the magnetic bedspread.

The invention will be now described by reference to the appended drawings.

Figure 1 is a perspective view of a magnetic bedspread.

Figure 2 is a cross-sectional view of a magnetic bedspread.

Figure 3 is a flow-chart showing the manufacturing steps of a magnetic sheet in accordance with this invention.

Figure 4 is an enlarged cross-sectional view showing the condition of coated surface of plastic magnet.

Figure 5 is a cross-sectional view showing the adhering processes of plastic magnets on the sheet material in accordance with the present invention.

Figure 6 is a perspective view of a magnetic mattress having a portion broken off.

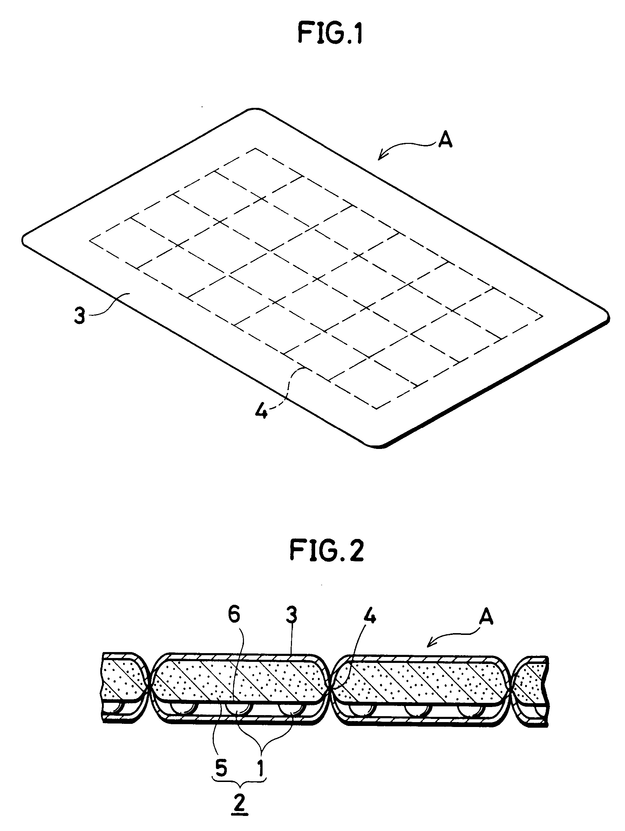

Figures 1 and 2 show a magnetic bedspread A, wherein a magnetic sheet 2 having mounted a multiple number of plastic magnets 1 is covered with a cloth 3 on both of its upper and lower sides and is applied with quilting process with thread 4 averting the plastic magnet portions.

The above-mentioned magnetic sheet 2 has a structure, wherein a flexible urethane foam sheet 5 is covered with a cover on its upper and lower sides and is adhered with a multiple number of plastic magnets 1 of hemispheric shape by means of hot-melt adhesives.

Each plastic magnet 1 is made of plastic magnetic material by magnetizing at the time of or after injection molding or compression molding of a synthetic resin base material such as nylon which has been blended with such magnetic powder as palium-ferrite or strontium-ferrite (for instance, "Compodic" by Dainippon Ink & Chemical, Inc.).

The hot-melt adhesive 6 is a multiplex adhesive made of a thermoplastic resin, adhesiveness promoting additive and wax, and if necessary, an anti-oxidant and some filler may be added. As its main thermoplastic ingredient, ethylene vinyl acetate copolymer and polyamide may be cited, and as adhesiveness promoting additive, rosin, rosin-derivatives and pinen type resin are used. As for wax, such as paraffin wax and microcrystalin wax are used.

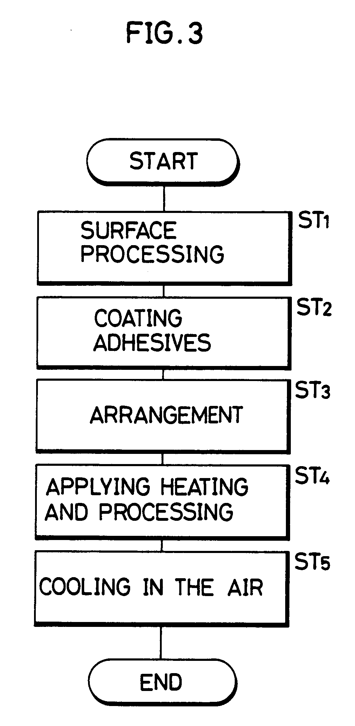

Figure 3 shows the order of manufacturing steps of the above-mentioned magnetic sheet in accordance with the present invention. In the step 1 of the chart (shown as STl), by applying the surface processing measure through the use of a predetermined chemical on the flat surface of the plastic magnet 1, an appropriate thickness of the resin base material is eroded to expose the surface of magnetic powder particles. As for the chemicals, such weak acid as phenol and ozone can be used besides strong acid like hydro-chloric acid and sulfuric acid. When this surface processing process is finished, melting the hot-melt adhesives by heating as the next step 2 is taken, and then the adhesives are coated on the roughened surface of the plastic magnets 1.

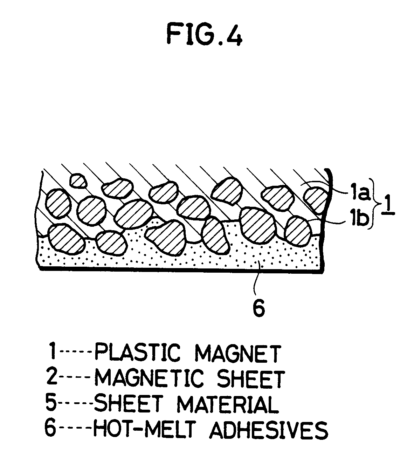

Figure 4 show enlarged condition of the plastic magnet 1 coated with adhesives. In the drawing, 1a and 1b show the resin base material and magnetic powder particles of the plastic magnet 1, and numeral 6 indicates the hot-melt adhesive respectively. According to the same drawing, the surface of the plastic magnets 1 is roughened at the eroded part of the resin base material 1a and exposed part of the magnetic powder particles 1b, and into this complicatedly eroded portion the hot-melt adhesive 6 has invaded, and the hot-melt adhesive 6 is solidified by natural air cooling after coating over the plastic magnet 1.

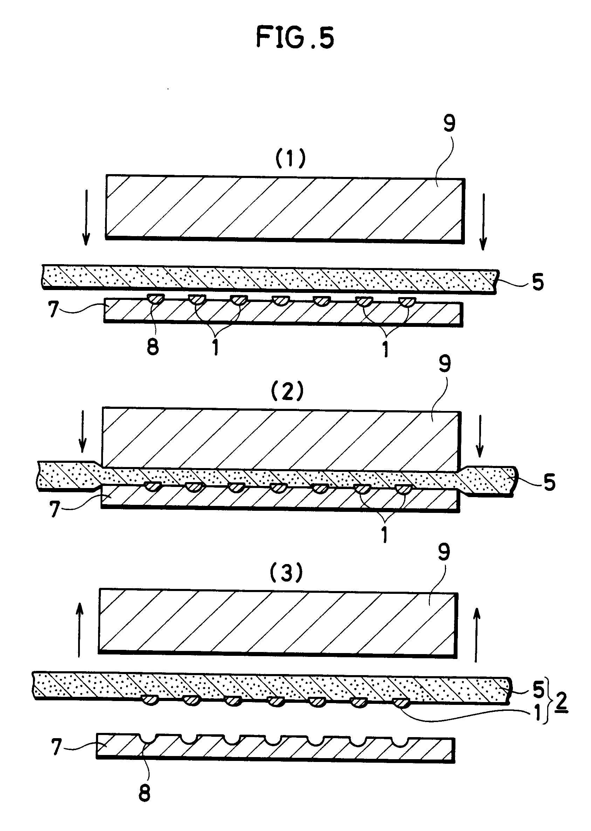

After finishing the coating process of the adhesives in step 2, it is taken over by step 3 wherein a plurality of plastic magnets 1 are placed in alignment on the metal support plate 7 as shown in figure 5(1) keeping the adhesive-coated surface to the upper side. This support plate 7 is provided with multiple recesses 8 of round dented shape on its upper surface so that those round shaped concave recesses 8 will accept hemispheric plastic magnets 1 to determine the positions of each plastic magnet 1.

After arranging the plastic magnets 1 in position, the sheet material 5 is placed on the support plate 7, over which a heating place 9 is let to come down. Said heating plate is heated to a temperature sufficient to melt the hot-melt adhesive. As shown in figure 5(2), by sandwiching the sheet material 5 with the heating plate 9 and the support plate 7, the sheet material 5 and each plastic magnet 1 are heat-pressed. This will heat and melt the hot-melt adhesive to make each plastic magnet 1 bonded with the sheet material 5. (Step 4)

Subsequently, when the heating plate is lifted, the hot-melt adhesive will be solidified by cooling down through natural air cooling, which brings each plastic magnet 1 bonded on the sheet material into one unit body, completing to make the magnetic sheet 2. (Step 5)

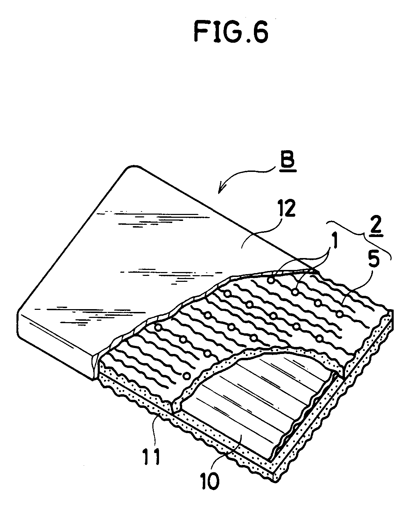

Figure 6 shows another example of application of said magnetic sheet 2.

There is shown a magnetic mattress B having a plastic corrugated plate 10 made of synthetic resin in the middle, sandwiched between a magnetic sheet 2 on the upper side and a cushioning sheet 11 on the lower side, the entire construction being enclosed in a cover sheet 12. The above-mentioned magnetic sheet 2 is formed of a structure, wherein a cushioning sheet 11 and the same sheet material 5 having mounted a multiple number of plastic magnets 1 on the surface by use of the hot-melt adhesives are used.

## Claims

1. A method of manufacturing a magnetic sheet (2) by adhering a plurality of plastic magnets (1) on a sheet material (5), comprising the steps of :
(a) surface processing the adhering surface of each plastic magnet (1) in preparation for adhesion onto the sheet material (5) by eroding and roughening with a chemical so as to expose magnetic powder particles,
(b) coating said roughened surface of each plastic magnet (1) with heat-melt adhesives (6), and
(c) heat-pressing the sheet material (5) and each plastic magnet (1) after applying the adhesive-coated surface of each plastic magnet (1) onto the surface of the sheet material.

## Patentansprüche

1. Verfahren zum Herstellen eines magnetischen Bettuchs (2) durch Anheften einer Mehrzahl von Kunststoffmagneten (1) auf ein Tuchmaterial (5), umfassend die Schritte des
(a) Oberflächenbehandelns der Haftfläche jedes Kunststoffmagneten (1) als Vorbereitung auf das Anheften auf das Tuchmaterial (5) durch Erodieren und Aufrauhen mittels einer Chemikalie, um so Magnetpulverpartikel freizulegen,
(b) Beschichtens der aufgerauhten Oberfläche jedes Kunststoffmagneten (1) mit Schmelzkleber (6) und
(c) Heißpressens des Tuchmaterials (5) und jedes Kunststoffmagneten (1) nach dem Aufbringen der mit Klebstoff beschichteten Oberfläche jedes Kunststoffmagneten (1) auf die Oberfläche des Tuchmaterials.

## Revendications

1. Procédé de fabrication d'une feuille magnétique (2) par collage d'une pluralité d'aimants plastiques (1) sur un matériau en feuille (5) comprenant les étapes consistant à :
(a) traiter en surface la surface de collage de chaque aimant plastique (1) pour préparer le collage sur le matériau en feuille (5) en l'érodant et la rendant rugueuse à l'aide d'un produit chimique, de manière à dénuder des particules de poudre magnétique,
(b) revêtir ladite surface rendue rugueuse de chaque aimant plastique (1) avec des adhésifs thermo-fondus (6), et
(c) presser à chaud le matériau en feuille (5) et chaque aimant plastique (1) après avoir appliqué la surface revêtue d'adhésif de chaque aimant plastique (1) sur la surface du matériau en feuille.
